# EUROPEAN PATENT APPLICATION

(11) **EP 4 450 035 A1**
(43) Date of publication of application: **23.10.2024**
(21) Application number: 23185001.7
(22) Date of filing: 12.07.2023
(51) Int. Cl.: A61F 13/15, A61F 13/532, A61F 13/533, A61F 13/534

(54) **ABSORBENT ARTICLE AND METHOD OF MAKING**

(30) Priority: 20.04.2023 EP 23168880
(71) Applicant: Ontex BV, 9255 Buggenhout (BE); Ontex Group NV, 9320 Erembodegem (BE)
(72) Inventor: Lambertz, Christina, 56566 Neuwied (DE)
(74) Representative: Macchetta, Andrea

(57) **Abstract**

An absorbent article comprising: a topsheet; a backsheet; and an absorbent core sandwiched between said topsheet and backsheet; said core comprising absorbent material that is substantially enclosed by a core wrap, wherein said core wrap comprises a top core wrap layer and a bottom core wrap layer, said core comprising an absorbent-material-deposition zone comprising a first basis weight of absorbent material, and wherein the top core wrap layer and a bottom core wrap layer are joined together at a plurality of discrete bonding regions substantially free of absorbent material; and wherein said core further comprises one or more unfastened regions comprising a second basis weight of absorbent material, and wherein the first basis weight is greater than the second basis weight; wherein the plurality of discrete bonding regions and the one or more unfastened regions are substantially connected to each other with at least a plurality of said discrete bonding regions (Bᵣ) being disposed outboard of a longitudinal centreline (y) of said absorbent core (4) and the discrete bonding regions are positioned such that neighbouring and/or consecutive bonding regions are separated by at least a portion of said absorbent-material-deposition zone along a longitudinal direction running substantially parallel to a longitudinal centreline of said absorbent core.

## Description

### TECHNICAL FIELD

The disclosure relates to absorbent articles such as disposable absorbent articles, preferably selected from the group consisting of diapers (whether for baby, youth, or adults), pants (whether for baby, youth or adults), briefs, pantyliners, slips, sanitary napkins or towels, sanitary napkins for light incontinence, and the like; preferably selected from diapers and pants.

### BACKGROUND

The disposable absorbent articles are articles which are used in proximity with the skin of a wearer to absorb and retain body exudates; they are typically constituted by an upper liquid-pervious topsheet, a bottom liquid impervious backsheet and an absorbent core disposed between them. The absorbent core of this kind of articles is generally composed of fibers of absorbent material and/or particles of superabsorbent material; its main functions are to absorb and to retain liquid or semi-liquid exudates from the human body that penetrate through the upper layer of the article. For the absorbent core to be effective, it desirably has a good integrity, that is to say, the absorbent core must maintain its structure and not break or collapse either in a dry or in a wet state. If the absorbent core breaks, its absorption and retention capacity as well as its ability to distributing liquids may be compromised and further increasing the risk of leakage, agglomerations of absorbent material and discomfort to the wearer. Multiple attempts have been made to prevent or minimize these shortcomings such as the use of nets, adhesives, core wraps, embossing and others.

Some examples that attempt to improve the integrity of the absorbent cores, include for example US Pat. No. 8, 198, 506 and US Pat No. 7,718,021 (Kimberly Clark Worldwide Inc.) which relate to a stabilized absorbent composite web and the method for producing it; said absorbent composite comprises a first sheet joined to a second sheet through a plurality of holes formed in the absorbent core. These patents are fully focused on improving the integrity of the absorbent core sacrificing the ability to absorb liquids more quickly as well as the ability to distribute them more evenly and efficiently through the absorbent core.

Other examples focus in improving the distribution of fluids in the absorbent core using channels. Articles comprising channelled cores are becoming more and more sought after in the market in view of their improved liquid distribution properties and reduction in amount of absorbent material used. Examples are described in the literature such as EP 3 342 386 A1, EP 3 692 963 A1, EP 3 542 766 A1, EP 3 738 562 A1 and the like. The preferred means of creating such channels is consistently by bonding directly upper and lower layers of an enveloping core wrap sheet in such a way to create channel-forming areas that are substantially free of absorbent material.

Other examples relate to absorbent cores free of cellulose fibers aiming at improving the containment and retention capacity of the core, as for example US20150342796A1 from Procter & Gamble which refers to an absorbent core comprising a core wrap enclosing superabsorbent particles: the superabsorbent particles form a pattern of dot-shape discrete areas which can be oriented longitudinally or transversely; these areas are separated from each other by areas substantially free of material and could also contain channels free of absorbent material.

Other examples relate to absorbent cores using zones having different basis weight and density within the core. Depending the way these zones are disposed, they are used for different purposes. For example, EP3238676B1 (Procter & Gamble) describes an absorbent core with first and second longitudinal channels free of absorbent material; the core may further comprise one or more transverse oriented folding lines in which the basis weight of absorbent material reaches a minimum relative to the immediately adjacent regions. These zones with lower basis weight are used for facilitating the folding and as they reach the longitudinal sides of the core.

Other attempts to overcome the above mentioned shortcomings are described for example in WO2021/123221 A1 that relates to a disposable absorbent article comprising a liquid-pervious topsheet, a liquid-impervious backsheet and an absorbent assembly between the topsheet and the backsheet. The absorbent assembly comprises an absorbent core, an upper core wrap and a lower core wrap. The absorbent core comprises at least two cavities free of absorbent material wherein the upper core wrap and the lower core wrap are permanently attached to each other, said cavities being interconnected via at least one connecting line comprising absorbent material. The absorbent core having a first basis weight in the zone out of the cavities and the connecting lines and a second basis weight in the connecting lines, the first basis weight being greater than the second basis weight. in the cavities.

There however still remains a need for absorbent articles that have excellent core integrity whilst maintaining fast liquid distribution and being simple and cost-effective to manufacture.

### SUMMARY

In a first aspect, the disclosure relates to an absorbent article comprising: a topsheet; a backsheet; and an absorbent core sandwiched between said topsheet and backsheet; said core comprising absorbent material that is substantially enclosed by a core wrap, wherein said core wrap comprises a top core wrap layer and a bottom core wrap layer, said core comprising an absorbent-material-deposition zone comprising a first basis weight of absorbent material, and wherein the top core wrap layer and a bottom core wrap layer are joined together at a plurality of discrete bonding regions substantially free of absorbent material; and wherein said core further comprises one or more unfastened regions comprising a second basis weight of absorbent material, and wherein the first basis weight is greater than the second basis weight; wherein the plurality of discrete bonding regions and the one or more unfastened regions are substantially connected to each other with at least a plurality of said discrete bonding regions (Bᵣ) being disposed outboard of a longitudinal centreline (y) of said absorbent core (4) typically such that said longitudinal centreline (y) is not substantially overlapped by said at least plurality of discrete bonding regions (Bᵣ), and the discrete bonding regions are positioned such that neighbouring and/or consecutive bonding regions are separated by at least a portion of said absorbent-material-deposition zone along a longitudinal direction running substantially parallel to a longitudinal centreline of said absorbent core.

In a second aspect, the disclosure relates to a method for the manufacture of absorbent articles, said method comprising the steps of: providing a first web of material, preferably a liquid permeable nonwoven; depositing absorbent material onto said first web at an absorbent-material-deposition zone and such that one or more areas of said first web material remain substantially void of absorbent material, said one or more areas corresponding to at least a plurality of discrete bonding regions, and wherein the absorbent-material-deposition zone comprises a first basis weight of absorbent material; providing a second web of material, preferably a liquid permeable nonwoven, and applying said second web of material over the deposited absorbent material, or folding said first web to enclose the deposited absorbent material therein; joining the first and second webs together, or the folded first web, at plurality of discrete bonding regions such that one or more unfastened regions are formed having a second basis weight of absorbent material; optionally sandwiching the absorbent core between a liquid permeable layer and a substantially liquid impermeable layer oppositely disposed; wherein the first basis weight is greater than the second basis weight and the plurality of discrete bonding regions are substantially connected to one or more unfastened regions and the discrete bonding regions are positioned such that neighbouring bonding regions are separated by at least a portion of said absorbent-material-deposition zone along a longitudinal direction running substantially parallel to a longitudinal centreline of said absorbent core.

### BRIEF DESCRIPTION OF THE FIGURES

**FIG. 1** is a plan view representation of an article according to an embodiment of the present disclosure.
**FIG. 2** is an enlargement of a portion A of Fig. 1.
**FIG. 3** is a plan view representation of an article according to an embodiment of the present disclosure.
**FIG. 4** is an exploded view representation of an article according to an embodiment of the present disclosure.
**FIG. 5** is a plan view representation of an article according to an embodiment of the present disclosure.
**FIG. 6** is a plan view representation of an article according to an embodiment of the present disclosure.
**FIG. 7** illustrates a channel formation according to an embodiment of the present disclosure, for example in articles of Figs. 5-6.
**FIG. 8** illustrates an equipment for the manufacture of an article according to an embodiment of the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-40% or less, or +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a, preferably unitary, structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within ±45° of the lateral direction.

The term "longitudinal" refers to a direction running parallel to the maximum linear dimension of the article and when combined with the term "substantially" includes directions within ±45° of the longitudinal direction.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

### THE ARTICLE

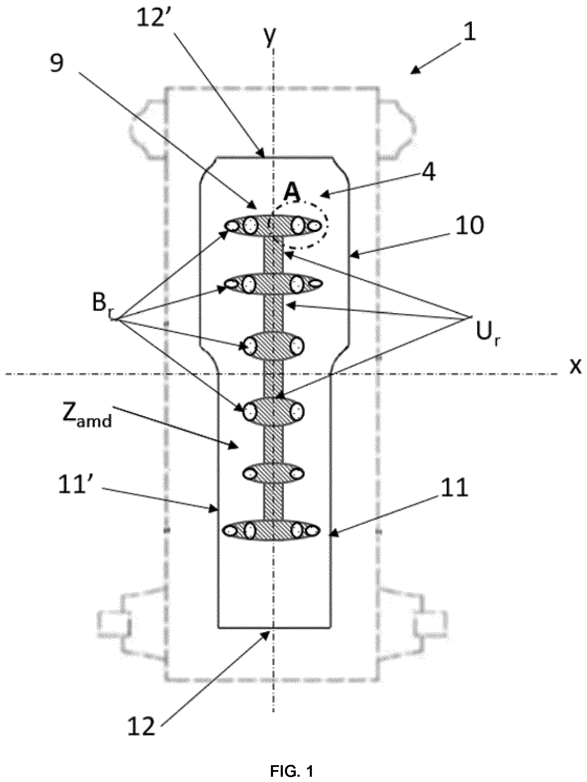

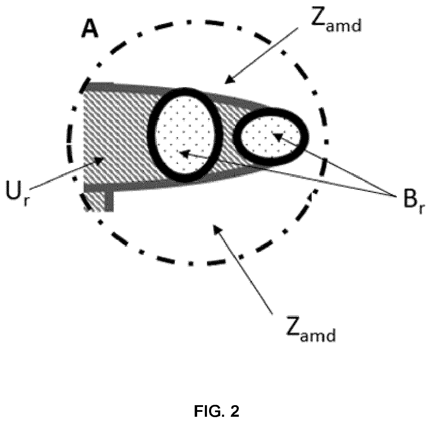

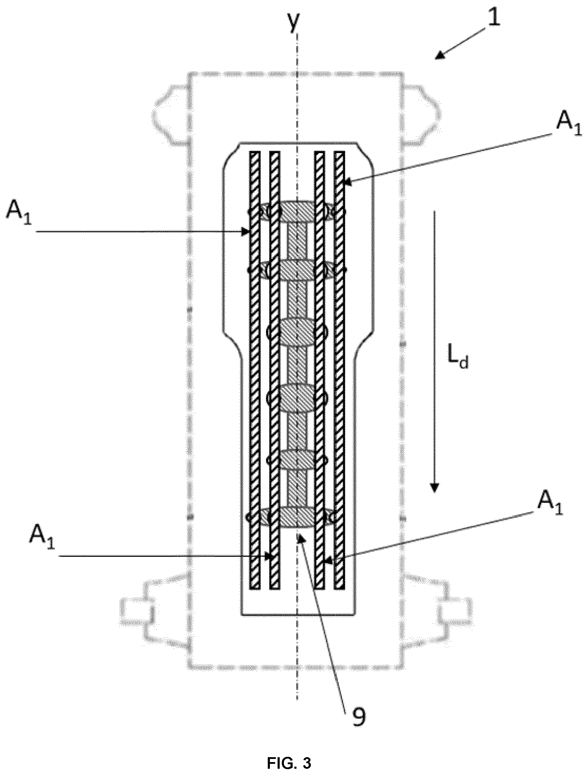

Absorbent articles (1) described herein, and as exemplified in Figs. 1-3, comprise: a topsheet (2); a backsheet (3); and an absorbent core (4) sandwiched between said topsheet (2) and backsheet (3); said core (4) typically comprises absorbent material (5) that is substantially enclosed by a core wrap (6), wherein said core wrap (6) may comprise a top core wrap layer (7) and a bottom core wrap layer (8), and wherein said top and bottom layers are typically joined together along one or more continuous or discontinuous attachment areas (or discrete bonding region(s)). The one or more continuous or discontinuous attachment areas may form one or more channels (9) typically together with or in conjunction with other structures (e.g. unbonded regions) as will be described in more detail herein below, the channels generally being substantially free of absorbent material. Channels described in any of the embodiments herein being generally positioned inboard of an outer perimeter (10) of said core (4) formed by two opposed lateral edges (11, 11') connecting two opposed transversal ends (12, 12') of said core (4).

The cores (4) herein comprising an absorbent-material-deposition zone (Zamd) comprising a first basis weight of absorbent material (5), and wherein the top core wrap layer (7) and a bottom core wrap layer (8) are joined together at a plurality of discrete bonding regions (Br) substantially free of absorbent material (for example the amount of absorbent material in the bonding region is less than about 5gsm, preferably less than about 3gsm, even more preferably less than about 2gsm, most preferably about 0 gsm). The bonding may be carried out by the use of adhesive or by mechanically bonding the layers together such as ultrasonic bonding, thermal bonding, combinations thereof and the like.

The cores (4) herein further comprise one or more unfastened regions (Ur) comprising a second basis weight of absorbent material (5), and wherein the first basis weight is greater than the second basis weight. The discrete bonding regions (Br) and the one or more unfastened regions (Ur) being substantially connected to each other (for example when viewed in a planar direction and/or collectively form a continuous shape or channel typically at least in dry state) with at least some (e.g. one or more, or a plurality), preferably the majority, even more preferably all, of said discrete bonding regions (Bᵣ) being disposed outboard of a longitudinal centreline (y) of said absorbent core (4) typically such that said longitudinal centreline (y) is not substantially overlapped by the said at least some discrete bonding regions (Bᵣ), and wherein the discrete bonding regions (Bᵣ) are positioned such that, preferably each, neighbouring and/or consecutive bonding regions (Bᵣ) are separated by at least a portion of said absorbent-material-deposition zone (Z_{amd}) along a longitudinal direction (L_{d}) running substantially parallel to a longitudinal centreline (y) of said absorbent core (4). Advantageously this allows to ensure good core integrity as well as maximised surface area coverage of liquid distribution via mass flow over an initial wetting period, yet permit volume expansion within cavities formed in the unfastened regions to accommodate neighbouring absorbent material as it swells whilst minimising the size of restriction areas formed at top/bottom core wrap joints. I may also further aid to reduce cost by permitting a reduction in amount of absorbent material yet retain a larger area for swelling capacity in wet state.

The one or more unfastened regions (Ur) may be substantially free of absorbent material generally meaning that the second basis weight may be less than about 5gsm, preferably less than about 3gsm, even more preferably less than about 2gsm, most preferably about 0 gsm. Advantageously, although no connection of top and bottom core wrap layers is present in such region the substantial absence of absorbent material (at least during the early wetting stages) in such region facilitates fluid transfer by mass flow between connecting bonding regions. Such may further permit maintaining the desired fluid distribution characteristics as well as accommodating absorbent material swelling whilst retaining core integrity in an effective manner.

The bonding regions (Bᵣ) may comprise permanent and/or temporary attachments. Typically, permanent attachments being arranged to remain substantially integral upon wetting and temporary attachments being arranged to release upon wetting. By wetting being intended exposing the said attachments to liquid for at least about 20 seconds, preferably at least about 30 seconds, more preferably at least about 50 seconds (typically at room conditions and with a liquid being at a body temperature of about 37 °C, the liquid may be a saline solution having 0.9% NaCl). For example, permanent attachments may comprise adhesive that is substantially liquid insoluble and temporary attachments may comprise adhesive that is substantially liquid soluble, or by other joining means e.g. by selective application of lower joining pressure, or contaminating the areas to be bonded with a dusting layer of absorbent, or the like generally forming a weaker attachment so that upon swelling of the absorbent material the force generated pulling the said layers apart is greater than the joining force provided by the joining means. Other examples of permanent attachments include mechanical bonding such as ultrasonic bonding, thermal and pressure bonding, and the like.

The bonding regions (Bᵣ) herein may comprise permanent and temporary attachments typically alternatingly disposed such that each temporary attachment is adjacent to, preferably interposed between, said temporary attachment(s); or vice versa. Advantageously this allows the formation of intermediate capillary acceleration zones allowing liquid to be transferred by capillary action especially during a second time period of wetting as described herein. Moreover, such temporary attachments permit to reduce the overall size (e.g. area and/or length) of the bonding regions especially during said second time period making a larger surface of the core available for wicking of liquid to maximise absorbent core usage.

Although the unfastened regions (Ur) may not be permanently (or physically/chemically) joined together, such regions may still comprise top and bottom core wrap layers (7, 8) in close proximity to each other (e.g. the layers may be adjacent or immediately adjacent to each other). This may be achieved for example by calendaring or otherwise compressing the absorbent core laminate after the step of enclosing the absorbent material therein as for example described in more detail herein below.

The absorbent article (1) may comprise a plurality of unfastened regions (Ur) connected to each other by the discrete bonding regions (Br), preferably wherein each said unfastened region (Ur) is positioned adjacent to at least one of the discrete bonding regions (Br). Advantageously, this permits to maximise the surface area of liquid distribution by mass flow during an initial wetting period.

The total length of the channel(s) (9) herein may be from 10% to 95%, preferably from 12% to 90%, more preferably from 15% to 85%, even more preferably from 20% to 80%, of a length of the absorbent core (4) generally said lengths taken along a longitudinal direction running substantially parallel to the longitudinal centerline (y).

At least portions of the unfastened regions (Ur) may be separated by the absorbent-material-deposition zone (Zamd) along at least an axis substantially parallel to the longitudinal centreline (y) and/or at least some of the discrete bonding regions (Bᵣ) may be separated by the absorbent-material-deposition zone (Z_{amd}) along at least an axis substantially parallel to the longitudinal centreline (y). Such arrangement may permit effective liquid distribution by wicking in areas of the core between unfastened regions.

Preferably, the unfastened regions (Uᵣ) are comprised in a plurality (i.e. a plurality of unfastened regions) and separated by discrete bonding regions (Bᵣ), preferably wherein said plurality of unfastened regions (Uᵣ) separated by discrete bonding regions (Bᵣ) are positioned in a front half and/or rear half of the absorbent core (4). The front half of the core may be positioned between a front transverse edge (12') and a transverse centerline (x), and the rear (or back) half of the absorbent core may be positioned between a rear transverse edge (12) and the transverse centerline (x). Advantageously risk of stiffening structures formed at the center of the core are reduced.

Preferably, the discrete bonding regions (Bᵣ) have an aspect ratio of from 0.4 to 3.0, preferably from 0.5 to 2.5, even more preferably of about 1. Advantageously this allows to limit the size of restriction areas continuously formed along top/bottom core wrap joints. Moreover, advantageously, this may permit each discrete bonding regions (Bᵣ) to not form a channel hence maximizing the available space for liquid absorption (yet together with unfastened regions yet being able to forming channel(s), in at least dry state and a first wetting state, to still allow for an initial liquid distribution by mass flow as described in more detain within embodiments herein).

Preferably, the discrete bonding regions (Bᵣ) are separated by one or more unfastened regions (Uᵣ) along an axis parallel to a transversal centerline (x) that is substantially perpendicular to the longitudinal centreline (y). Advantageously, this may allow greater absorbent material restriction to movement along the core in the longitudinal direction in front/rear halves of the core towards the center or vice-versa and hence contributing to core integrity. Moreover, this arrangement may aid guiding of absorbent material into the cavity of the unfastened regions during wetting whilst swelling of absorbent material in neighbouring areas within the absorbent deposition zone occurs.

The size (e.g. width-wise dimension) of the unfastened regions (Ur) positioned between discrete bonding regions (Bᵣ) taken along an axis parallel to a transversal centerline (x) may be from about 3mm to about 50mm, preferably from about 5mm to about 40mm, even more preferably from about 10mm to about 30mm.

Preferably, as for example shown in Figs/ 1-3, the discrete bonding regions (Bᵣ) are positioned outboard of a longitudinal centreline (y) of said absorbent core (4) such that said longitudinal centreline (y) is not substantially overlapped by said discrete bonding regions (Bᵣ). Advantageously this may allow maximising absorption capacity and free-swelling mechanisms in the central region of the core.

The ratio of first basis weight to second basis weight (i.e. first basis weight / second basis weight) may be greater than about 1.5, preferably greater than about 2, more preferably greater than 3, even more preferably from greater than about 4, even more preferably greater than about 5. Advantageously maintaining the amount of absorbent material in the unfastened regions significantly lower than neighbouring amounts in the deposition area allows for added flexibility of the core and article as it is able to better conform to the body especially upon movement thanks to the formation of hinges or folding lines along such positions, and moreover limits the formation of blockages between the bonding regions that may obstruct liquid distribution along the unfastened regions by mass flow at least during a first wetting period as described in more detail herein.

Preferably, the top core wrap layer (7) or bottom core wrap layer (8) comprises one or more stripes of first adhesive (A1) that may be continuous or discontinuous. Preferably, the stripes of adhesive may have an aspect ratio of greater than 1, preferably greater or equal to 1.5, even more preferably from 2 to 160. The stripes generally extending along an axis substantially parallel to the longitudinal centreline (y), and generally wherein at least one, preferably at least two, of said stripes overlaps with one or more, preferably a plurality, of the discrete bonding region(s) (Br). The stripes being preferably arranged such that each of said stripes does not substantially overlap (for example overlaps less than 20% of its width or area, preferably about 0% of its width or area) with the one or more unfastened regions (Ur), preferably wherein each said stripe overlaps at least a portion of the absorbent-material-deposition zone (Zamd). Advantageously this allows to form bonding region(s) and unfastened regions in an effective manner without compromising production speed and yet provide some resistance to movement of the absorbent material also within the absorbent-material-deposition zone.

Preferably, the opposing bottom core wrap layer (8) or top core wrap layer (7) is substantially free of adhesive at least in areas that are congruent (typically meaning herein "coinciding when superimposed") with the stripes of first adhesive. Advantageously this reduces risk of contamination by residual absorbent material sticking to the bonding region that may impact joining strength and hence also core integrity.

Preferably, the absorbent core comprises a left longitudinal half and right longitudinal half running along a length of said core and being adjacent to each other with the longitudinal centerline (y) being interposed therebetween, and wherein said plurality of stripes comprise at least two, preferably spaced apart, stripes that are positioned on said left longitudinal half and/or right longitudinal half.

Neighbouring (e.g. ones that are closest to each other e.g. directly neighbouring) bonding regions (Bᵣ) are free of unfastened regions (Uᵣ) in direct connection therewith along the longitudinal direction (L_{d}). Advantageously, this may allow avoiding an unfastened region in between and adjacent two consecutive bonding regions in the longitudinal direction and hence permit hinge structures aiding cup formation during wetting to be formed for better comfort as well as increasing area available for absorption.

The unfastened regions (Ur) and the discrete bonding regions (Br) collectively may form one or more channels (9) suitable for guiding liquid via mass flow (for example similar to flow of liquid along a duct) therethrough and/or therealong during a first time period, and wherein one or more portions of said channel(s) corresponding to the unfastened regions (Ur) open (for example top and bottom core wrap layers move further away from each other to increase a cavity volume therebetween) to further accommodate absorbent material (5) therein as adjacent absorbent material (5) of the absorbent-material-deposition zone (Zamd) swells. Advantageously optimised core usage also during subsequent wetting(s) may be attained.

The unfastened regions (Ur) may be suitable for guiding liquid by capillary flow (for example by wicking of liquid between narrow spaces within the absorbent material) at least during a second time period for example once absorbent material enters the cavity formed by said unfastened regions (Ur) during swelling of the absorbent material in neighbouring areas of the absorbent-material-deposition zone. Advantageously this may permit liquid absorption also against gravity and/or movement of the subject especially during an already wetted state.

The unfastened regions (Uᵣ) may comprise an amount (generally a small amount, e.g. less than 2g, preferably less than 1g) of absorbent material that is joined to at least one of the top and bottom core wrap layers typically by adhesive. This may be achieved by application of e.g. adhesive prior to absorbent material deposition explained in some more detail hereinbelow with reference to process and equipment embodiments.

The absorbent material may be at least partially immobilised onto at least one of the top and bottom core wrap layers typically by adhesive within the unfastened regions (Uᵣ) and/or the unfastened regions (Uᵣ) may be purposively contaminated with said absorbent material; and may serve to actively force the top and bottom core wrap layers to separate and move further away from each other during swelling of absorbent material generally upon wetting. As top and bottom core wrap layers are moved apart a cavity may be formed, the free-volume of which may be filled with absorbent material located in areas neighbouring the unfastened regions.

Preferably the second time period occurs after the first time period; and/or the second time period is greater than the first time period; and/or the second time period is more than 15 seconds, preferably more than 20 seconds (for example from 25 seconds to 2 minutes, preferably from 30 seconds to 1 minute). Advantageously this permits delayed activity of capillary flow after an initial mass flow occurrence such to minimise slowing down of liquid during first wetting along the core.

The top and bottom core wrap layers (7, 8) are preferably joined together by one or more adhesives and/or mechanical bonds. Advantageously this permits not only to attain excellent liquid transfer performance but further also aids to retain core integrity particularly during wetting and swelling of the absorbent material. This may be the case at the bonding region(s) (Br) herein and/or in other regions of the core such as the peripheral edges so as to seal and enclose the absorbent material to prevent lateral outflow thereof.

In an embodiment, the one or more channels (9) have a length along a longitudinal axis (y) that is less than a length of the absorbent core (4) extending along said axis (y) such that the one or more channels (9) do not extend to the perimeter (10) of the core (4). Advantageously this aids to limit risk of undesirable leakage from the edges of the core and article.

The channels (9) suitable herein may have different shapes and sizes depending on the needs.

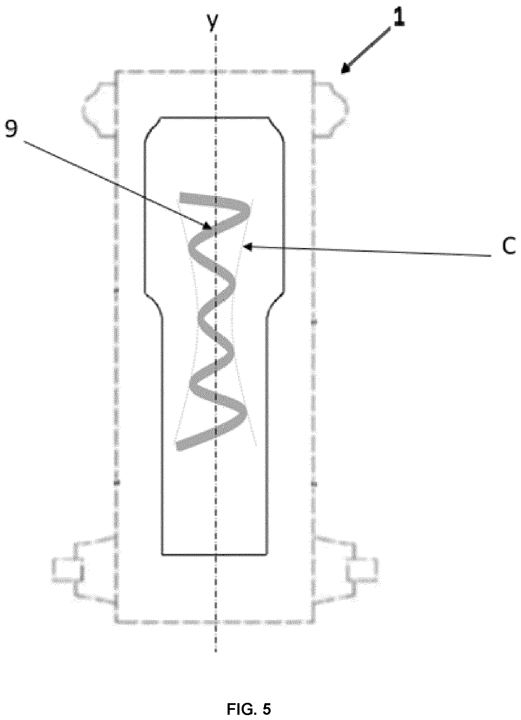

As for example illustrated in Fig. 5, the unfastened regions (Ur) and the discrete bonding regions (Br) may collectively form a channel (9) having a curved shape, preferably comprising a meandering pattern. The curved shape may comprise a plurality of turning points (or positions), generally each turning point forming an apex of a curve, and wherein each said turning point is positioned along one or more imaginary lines (c) extending along a longitudinal direction being substantially parallel to the longitudinal centerline (y). The turning points may be positioned along a plurality of said imaginary lines (for example at least two). Said imaginary line(s) (c) may be substantially arc-shaped and may be separated from each other in a width-wise direction running substantially perpendicular to the longitudinal centerline (y) and may be positioned outboard (or on either side) of said longitudinal centerline (y). The turning points may follow a curved contour along said imaginary line(s) (c).

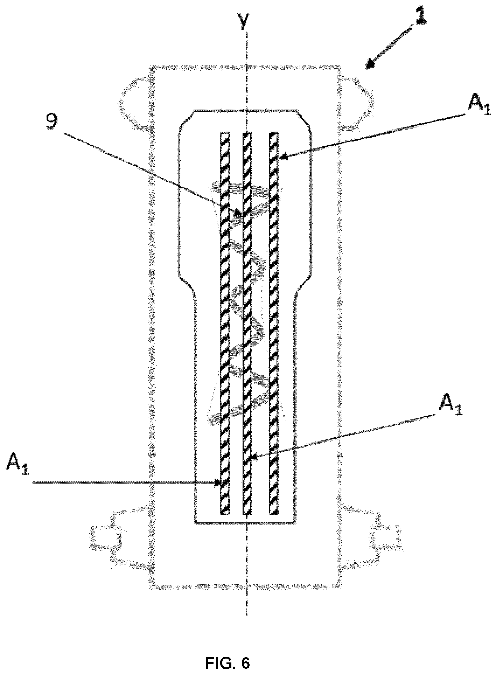

As for example illustrated in Fig. 6, the top core wrap layer (7) or bottom core wrap layer (8) may comprise one or more (preferably a plurality) of stripes of first adhesive (A1) typically as described herein-above such to adhere the top and bottom core wrap layers together at the discrete bonding regions (Br).

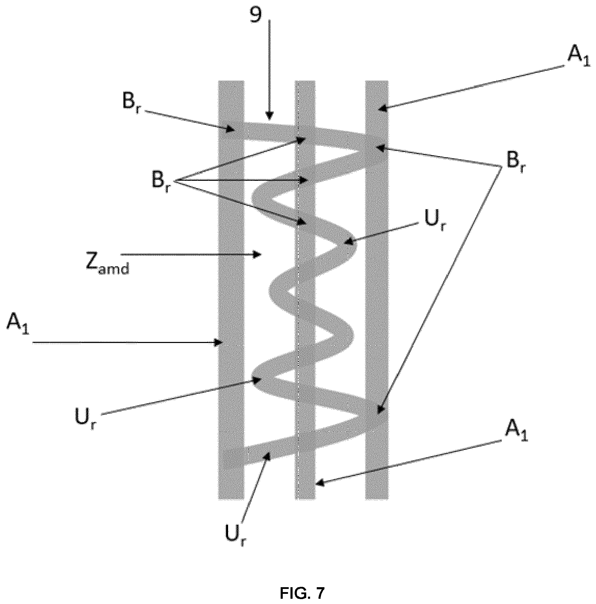

As for example illustrated in Fig. 7, the article may comprise a plurality of unfastened regions (Ur) and/or a plurality of discrete bonding regions (Br) that collectively form a channel (9).

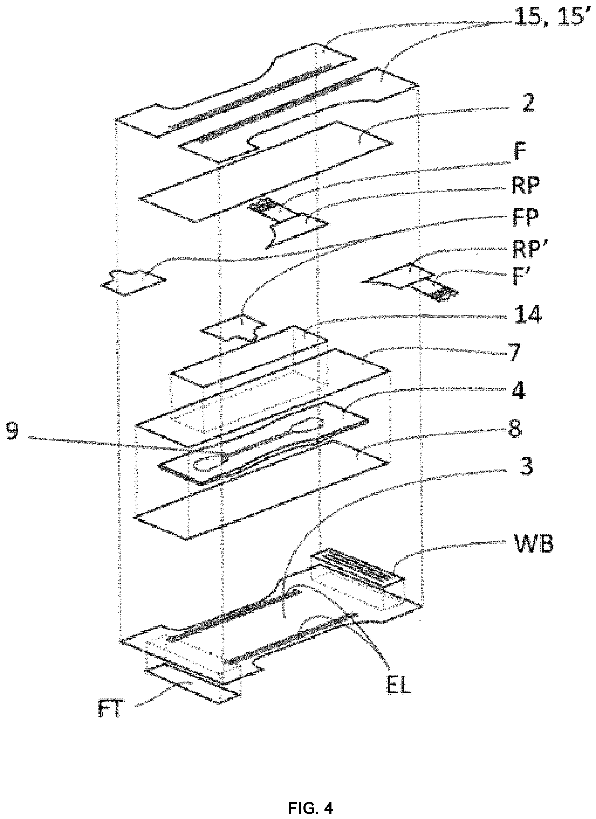

Advantages of the configurations exemplified in Figs. 1-7 include for example the possibility of forming channels via a single and/or continuous insert (as generally described herein-below) which can easily be mounted onto pockets of a rotating drum (as generally described herein-below) and further allows even pressure to be applied by pressure rollers (such as generally described herein-below) that can continuously glide over an evenly elevated surface hence increasing lifespan of components and allowing operation at higher speeds. In contrast, using multiple distinct inserts may not only increase complexity in accurate mounting and changeover time increase, but further included multiple discrete elevated zones so that the pressure roller(s) are forced to bounce up and down, leading to higher variability in the process and greater wear & tear.

Preferably, the unfastened regions (Ur) and the discrete bonding regions (Br) collectively correspond to a shape of an insert (sometimes herein referred to as non-porous insert) generally according to equipment and methods described herein-below.

In an embodiment, the article (1) further comprises a liquid distribution layer (14) positioned between the topsheet (2) and the absorbent core (4), the said layer may be any suitable nonwoven layer (including multi-layer laminates). Preferably the liquid distribution layer is a nonwoven layer selected from the group consisting of a spunbond nonwoven, carded nonwoven, spunlace nonwoven, and combinations thereof. The basis weight of the liquid distribution layer (14) is preferably from 15 gsm (g/m2) to 45 gsm (g/m2), preferably from 18 gsm (g/m2) to 40 gsm (g/m2), even more preferably from 20 gsm (g/m2) to 38 gsm (g/m2).

Preferably, the absorbent material (5) comprises superabsorbent polymer particles and/or cellulose fibers. The superabsorbent polymer particles may be comprised in an amount of from 60%wt to 100%wt, preferably from 70%wt to 90%wt, even more preferably from 75%wt to 85%wt, by total weight of the absorbent material. Having some cellulose fiber content may be advantageous in providing core integrity whilst limiting the amount of adhesive used as well as aid capillary flow effects described herein.

The article (1) herein may be disposable and selected from the group consisting of diapers, pants, briefs, pantyliners, slips, sanitary napkins and towels, and combinations thereof, preferably diapers or pants (for baby, youth, and/or adult subjects).

The article (1) herein may further comprise oppositely disposed lateral cuffs (15, 15') that generally act as longitudinal barriers to liquid leakage. The cuffs typically comprise a hydrophobic nonwoven layer and one or more elastics proximal to an apex thereof to form stand-up gathers. For clarity of the drawings, only one or two elastic strands are shown for each cuff, but typically each cuff may comprise from 1 to 4 elastic strands. The absorbent article may also comprise other typical components such as a back elastic waist feature (WB), a front elastic waist feature, frontal tape or landing zone (FT), leg elastics (EL) transverse barrier cuffs, front panels (FP), rear panels (RP, RP') that may be elastic and may comprise fasteners (F, F') for joining to the frontal tape, a wetness indicator that changes colour when contacted with urine, etc.

### THE PROCESS

A method for the manufacture of absorbent articles herein comprises the steps of: providing a first web of material (NW1), preferably a liquid permeable nonwoven; depositing absorbent material onto said first web at an absorbent-material-deposition zone (Z_{amd}) and such that one or more areas of said first web material (NW1) remain substantially void of absorbent material, said one or more areas corresponding to at least a plurality of discrete bonding regions (Bᵣ), and wherein the absorbent-material-deposition zone (Z_{amd}) comprises a first basis weight of absorbent material; providing a second web of material (NW2), preferably a liquid permeable nonwoven, and applying said second web of material (NW2) over the deposited absorbent material, or folding said first web (NW1) to enclose the deposited absorbent material therein; joining the first and second webs together, or the folded first web, at plurality of discrete bonding regions (Bᵣ) such that one or more unfastened regions (Uᵣ) are formed having a second basis weight of absorbent material; optionally sandwiching the absorbent core between a liquid permeable layer and a substantially liquid impermeable layer (typically corresponding to the topsheet and backsheet respectively) oppositely disposed; wherein the first basis weight is greater than the second basis weight and the plurality of discrete bonding regions (Bᵣ) are substantially connected to one or more unfastened regions (Uᵣ) with said discrete bonding regions (Bᵣ) being disposed outboard of a longitudinal centreline (y) of said absorbent core (4) such that said longitudinal centreline (y) is not substantially overlapped by said discrete bonding regions (Bᵣ) and the discrete bonding regions (Bᵣ) are positioned such that, preferably each, neighbouring bonding regions (Bᵣ) are separated by at least a portion of said absorbent-material-deposition zone (Z_{amd}) along a longitudinal direction (L_{d}) running substantially parallel to a longitudinal centreline of said absorbent core (generally the longitudinal centerline extending parallel to a machine direction). Preferably, the unfastened regions (Ur) and the discrete bonding regions (Br) collectively form one or more channels (9), preferably a single channel.

The method may comprise the step of applying one or more, preferably a plurality, of, preferably spaced apart, stripes of first adhesive on the second web of material (NW2) such that said adhesive overlaps the discrete bonding regions (Br) and substantially does not overlap the one or more unfastened regions (Ur). Advantageously this allows to ensure good bonding in the elongate bonding region and limit inadvertent bonding of the unfastened regions.

Preferably the method comprises the step of applying a plurality of, preferably spaced apart, stripes of first adhesive on the second web of material (NW2), said stripes being offset from a centerline of said second web (generally extending along a machine direction and corresponding to the longitudinal centerline y) such that they overlap the discrete bonding regions (Bᵣ) and do not substantially overlap the one or more unfastened regions (Uᵣ). At least portions of the absorbent-material-deposition zone (Zamd) may be overlapped by said adhesive.

The method may comprise the step of applying one or more stripes of second adhesive on the first web of material (NW1) such that said second adhesive does not overlap the discrete bonding regions (Br); and/or such that said second adhesive substantially does not overlap the one or more unfastened regions (Ur). Advantageously this limits risks of contamination by absorbent material sticking onto the first web and weakening the bonding strength.

The method may alternatively comprise the step of applying one or more stripes of second adhesive on the first web of material (NW1) such that said second adhesive does not overlap the elongate bonding region (Bᵣ); and such that said second adhesive does overlap the one or more unfastened regions (Uᵣ). Advantageously this may allow reduced contamination in the bonding region but does ensure some absorbent material to be adhered/contained within the one or more unfastened regions (Uᵣ) so that no to little bonding of the top/bottom core wrap layers occurs in such regions which may aid the provision of region(s) where such core wraps are forced away from each other during swelling of the absorbent material and hence actively create space for neighbouring absorbent material (e.g. within the absorbent material deposition zone) to fill the cavity and/or increased volume created.

In a preferred embodiment, the absorbent-material-deposition zone substantially corresponds to a suction zone (preferably as described herein) and the one or more unfastened regions (Ur) together with the at least one said discrete bonding regions (Br) substantially correspond to a non-suction zone (preferably as described herein).

The non-suction zone preferably being in the form of a non-porous insert having a shape corresponding to the shape of the one or more unfastened regions (Ur) and at least one said discrete bonding region (Br) (preferably all said discrete bonding region(s) (Br)) together.

In an embodiment, the method comprises the step of joining the first and second webs together, or the folded first web, at the one or more attachment areas (generally corresponding to the bonding regions herein) to form an absorbent core having one or more channels substantially free of absorbent material and at least in part flanked by absorbent material; preferably wherein the first and second webs, or the folded first web, are joined at the one or more attachment areas by one or more adhesives and/or mechanical bonds.

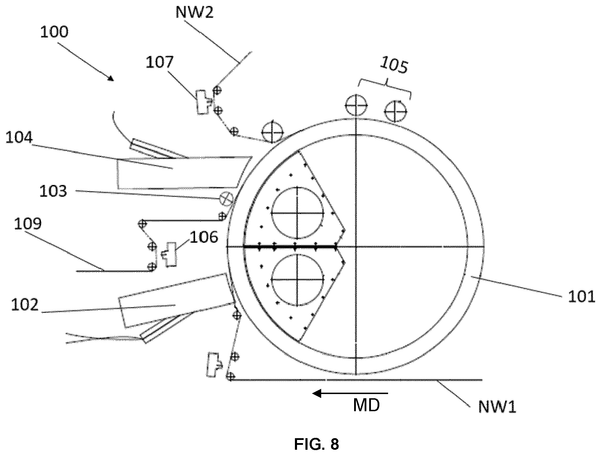

In an embodiment, exemplified in Fig. 8, the method for making an absorbent article comprising the steps of: i. providing a pocket comprising a porous cavity, wherein said cavity is in fluid communication with an under-pressure source; ii. providing a first nonwoven web (NW1) in the form of a bottom layer of a core wrap; iii. depositing said bottom layer onto said pocket; iv. depositing a first absorbent material (typically via an airstream, e.g. blowing but other means such as gravitational or printing may be suitable), comprising cellulose fibers and/or superabsorbent polymer particles, over at least a portion of a surface of said bottom layer; v. optionally depositing an intermediate layer (109) over the first absorbent material such that said first absorbent material is sandwiched between said intermediate layer (109) and bottom layer; vi. optionally joining said intermediate layer (109) to said bottom layer at one or more first distinct positions; vii. optionally depositing a second absorbent material (typically via an airstream, e.g. blowing), comprising cellulose fibers and/or superabsorbent polymer particles, over at least a portion of a said intermediate layer (109); viii. depositing a second nonwoven web (NW2) in the form of a top layer of a core wrap over the second absorbent material such that said second absorbent material is sandwiched between said top layer and optionally said intermediate layer (109); ix. joining said top layer and bottom layer, and optionally said intermediate layer (109), at one or more second distinct positions, to form an absorbent core; x. optionally joining an acquisition distribution layer to the body facing surface of said top layer, and preferably laminating the absorbent core and the acquisition distribution layer between a liquid pervious topsheet and a liquid impervious backsheet.

In an embodiment, the method further comprises the step of locally removing the absorbent material applied on said bottom and/or intermediate layers, preferably by a mechanical removal means preferably comprising one or more roller brush or air blower.

In an embodiment, the method further comprises the step of applying a first adhesive pattern on a surface of the intermediate layer facing the first absorbent material; and applying an second adhesive pattern on a surface of the top layer facing the second absorbent material, preferably wherein the first and second patterns are substantially the same or different.

In one embodiment, the joining step(s) comprises the step of applying a pressure and/or adhering force to join the first, third, and when present second, sheet materials (as described herein) respectively, substantially concurrently with a suction force within the at least one suction zone typically such to combine a downward push with a substantially simultaneous downward pull wherein downward is the direction from the position at which pressure is applied to or towards the supporting member. This is typically achieved by ensuring that the attachment unit(s) herein is/are disposed such to superpose a vacuum region (V) within the support unit (typically in the form of a rotating drum). Advantageously this allows to attain good and strong adhesion without having to apply excessive pressures with the use of e.g. pressure rollers (or embossing rollers) that may inadvertently damage sections of the absorbent core.

In a preferred embodiment, the apparatus (100) for making absorbent articles herein comprises: a supporting member (101), generally in the form of a rotating drum, for supporting a first sheet material (being the bottom core wrap layer (8) along a surface thereof, typically said supporting member (101) comprising a plurality of said pockets generally disposed along a circumference thereof, wherein the surface of said supporting member (101) is provided with at least one suction zone and at least one non-suction zone;
a first application unit (102) configured for applying a first absorbent material (typically by depositing absorbent material via an airstream, e.g. blowing or otherwise) on said first sheet material on the supporting member (101); said first absorbent material being applied such that said first absorbent material is located on a portion of the first sheet material corresponding to the at least one suction zone, and wherein substantially no absorbent material is present on other one or more portions of the first sheet material corresponding to the at least one non-suction zone typically on at least one first attachment portion (herein also attachment zone or area or bonding region(s)); optionally a first sheet feed unit configured for applying a second sheet material (being the intermediate layer (109)) on top of the first absorbent material on the first sheet material; optionally a first attachment unit (103) configured for attaching said first sheet material to said second sheet material at least in the at least one first attachment portion; optionally a second application unit (104) configured for applying a second absorbent material (typically by depositing absorbent material via an airstream, e.g. blowing or otherwise) on said second sheet material and/or first absorbent material; said second absorbent material being applied such that said second absorbent material is located on a portion of the second sheet material, and wherein substantially no absorbent material is present on other one or more portions of the second sheet material on at least one second attachment portion; a second sheet feed unit configured for applying a third sheet material (being the top core wrap layer) on top of the first absorbent material or if present the second absorbent material on the second sheet material; a second attachment unit (105) configured for attaching said third sheet material to said first sheet material, or when present the second sheet material, at least in the discrete bonding regions (Br).

Preferably, the non-suction suction zone herein has a shape that corresponds to the channel(s) herein and/or the sum of the bonding regions (Br) and unfastened regions (Ur) herein.

In a preferred embodiment, at least the first application unit (102) (but preferably all the application units described herein) comprises a blowing means (such as an air stream source) to direct the absorbent material onto the bottom layer/first sheet material and/or the intermediate layer/second sheet material, and is preferably a non-contact application unit in that it is free of a rotatable laying-out roller comprising a plurality of pockets for applying a spread of absorbent material (also conventionally known or referred to as absorbent material printing). Especially when the absorbent material comprises cellulose fibers it is desirable to apply such absorbent material via a non-contact application in order to achieve good mixing and spacing apart of the superabsorbent polymer particles between cellulose fibers and limit agglomeration of said particles, this allowing to achieve cores with better liquid distribution properties along and across its surface.

In an embodiment, the supporting member (101) is a rotating drum and the at least one non-suction zone comprises at least one elongate zone extending in a circumferential direction of the rotating drum, a plurality of said non-suction zones may be comprised in said pocket. Preferably, the at least one non-suction zone is formed by at least one element being substantially planar with an outer surface of the rotating drum; and wherein the at least one suction zone is formed by at least one cavity comprising a substantially porous base that is positioned at a first radial distance from the center of said rotating drum being less than a second radial distance of said outer surface from said center.

In an embodiment, the apparatus further comprises a removing unit comprising a mechanical removal means configured for locally removing the absorbent material applied on said first and/or second sheet material above the at least one non-suction zone. Typically, wherein the mechanical removal means comprises one or more roller brush or air blower.

Preferably, the first sheet feed unit is positioned between the first application unit and the second application unit, and typically upstream of the first attachment unit (103) along a machine direction (MD). Advantageously this allows to ensure reduced risk of contamination between layers.

The apparatus may further comprise a first adhesive unit (106) arranged to apply an adhesive pattern on a surface of the second sheet material facing the first absorbent material.

Preferably, the apparatus comprises a second adhesive unit (107) arranged to apply an adhesive pattern on a surface of the third sheet material facing the first absorbent material, or when present the second absorbent material.

The first and/or second adhesive units may positioned upstream of said first and/or second attachment units (103, 105) respectively. The apparatus may further comprise a further adhesive unit arranged to apply an adhesive pattern on a surface of the first sheet material facing the first absorbent material in areas of corresponding to the absorbent-material-deposition zone described herein and in such a way to not overlap the one or more elongate bonding regions (Br).

In an embodiment, the first and/or second attachment units comprise a pressure means, such as a pressure roller (typically having a substantially smooth contact surface generally such that it is free of protrusions pressing into the channel forming areas so as to limit the need for process registration), arranged to provide an adhering force to join the first, second, and third sheet materials respectively; and preferably wherein the first attachment unit comprises a single pressure means, and the second attachment unit comprises a plurality, preferably from 2 to 5, of pressure means. It is understood herein that in absence of an intermediate layer (or second sheet material) a similar pressure roller arrangement may be used for applying a pressure directly to the laminate formed after placing the third sheet material (or second nonwoven web NW2) onto the absorbent material and the first sheet material (or first nonwoven web NW1).

In an embodiment, the first attachment unit is positioned between the first application unit and optional second application unit, and downstream of the first sheet feed unit, typically along a machine direction (MD). Advantageously this allows to ensure optimal adhesion and reduced risk of contamination between layers.

It is supposed that the present invention is not restricted to any form of realization described previously and that some modifications can be added to the presented embodiments without reappraisal of the appended claims.

## Claims

1. An absorbent article (1) comprising:
a topsheet (2);
a backsheet (3); and
an absorbent core (4) sandwiched between said topsheet (2) and backsheet (3);
said core (4) comprising absorbent material (5) that is substantially enclosed by a core wrap (6), wherein said core wrap (6) comprises a top core wrap layer (7) and a bottom core wrap layer (8), said core (4) comprising an absorbent-material-deposition zone (Z_{amd}) comprising a first basis weight of absorbent material (5), and wherein the top core wrap layer (7) and a bottom core wrap layer (8) are joined together at a plurality of discrete bonding regions (Bᵣ) substantially free of absorbent material; and wherein said core (4) further comprises one or more unfastened regions (Uᵣ) comprising a second basis weight of absorbent material (5), and wherein the first basis weight is greater than the second basis weight; **characterised in that** the plurality of discrete bonding regions (Bᵣ) and the one or more unfastened regions (Uᵣ) are substantially connected to each other with a plurality of said discrete bonding regions (Bᵣ) being disposed outboard of a longitudinal centreline (y) of said absorbent core (4) and **in that** the discrete bonding regions (Bᵣ) are positioned such that neighbouring and/or consecutive bonding regions (Bᵣ) are separated by at least a portion of said absorbent-material-deposition zone (Z_{amd}) along a longitudinal direction (L_{d}) running substantially parallel to a longitudinal centreline (y) of said absorbent core (4).

2. An absorbent article (1) according to Claim 1 comprising a plurality of unfastened regions (Uᵣ) separated by discrete bonding regions (Bᵣ), preferably wherein said plurality of unfastened regions (Uᵣ) separated by discrete bonding regions (Bᵣ) are positioned in a front half and/or rear half of the absorbent core (4).

3. An absorbent article (1) according to any of the preceding Claims wherein the discrete bonding regions (Bᵣ) have an aspect ratio of from 0.4 to 3.0, preferably from 0.5 to 2.5, even more preferably of about 1.

4. An absorbent article (1) according to any of the preceding Claims wherein the discrete bonding regions (Bᵣ) are separated by one or more unfastened regions (Uᵣ) along an axis parallel to a transversal centerline (x) that is substantially perpendicular to the longitudinal centreline (y).

5. An absorbent article (1) according to any of the preceding Claims wherein the discrete bonding regions (Bᵣ) are separated by the absorbent-material-deposition zone (Z_{amd}) along at least an axis substantially parallel to the longitudinal centreline (y).

6. An absorbent article (1) according to any of the preceding Claims wherein ratio of first basis weight to second basis weight is greater than about 1.5, preferably greater than about 2, more preferably greater than 3, even more preferably greater than about 4, even more preferably greater than about 5.

7. An absorbent article (1) according to any of the preceding Claims wherein the top core wrap layer (7) or bottom core wrap layer (8) comprises a plurality of, preferably spaced apart, stripes of first adhesive (A1) extending along an axis substantially parallel to the longitudinal centreline (y), and wherein each said stripe overlaps with at least one, preferably at least two, of the plurality of discrete bonding regions (Bᵣ), and said stripes arranged such that each of said stripes does not substantially overlap with the one or more unfastened regions (Uᵣ), preferably wherein each said stripe overlaps at least a portion of the absorbent-material-deposition zone (Z_{amd}).

8. An absorbent article (1) according to Claim 7 wherein the opposing bottom core wrap layer (8) or top core wrap layer (7) is free of adhesive at least in areas that are congruent with the stripes of first adhesive.

9. An absorbent article (1) according to any of Claims 7 to 8 wherein the absorbent core comprises a left longitudinal half and right longitudinal half running along a length of said core and being adjacent to each other with the longitudinal centerline (y) being interposed therebetween, and wherein said plurality of stripes comprise at least two, preferably spaced apart, stripes that are positioned on said left longitudinal half and/or right longitudinal half.

10. An absorbent article (1) according to any of the preceding Claims wherein neighbouring bonding regions (Bᵣ) are free of unfastened regions (Uᵣ) in direct connection therewith along the longitudinal direction (L_{d}).

11. An absorbent article (1) according to any of the preceding Claims wherein the unfastened regions (Uᵣ) and the discrete bonding regions (Bᵣ) collectively form one or more channels (9) suitable for guiding liquid via mass flow therethrough during a first time period, and wherein one or more portions of said channel(s) corresponding to the unfastened regions (Uᵣ) open to further accommodate absorbent material (5) therein as adjacent absorbent material (5) of the absorbent-material-deposition zone (Z_{amd}) swells.

12. An absorbent article (1) according to any of the preceding Claims wherein the unfastened regions (Uᵣ) are suitable for guiding liquid by capillary flow at least during a second time period.

13. An absorbent article (1) according to any of Claims 11 to 12 wherein the second time period occurs after the first time period; and/or wherein the second time period is greater than the first time period; and/or wherein the second time period is more than 15 seconds, preferably more than 20 seconds.

14. A method for the manufacture of absorbent articles, said method comprising the steps of:
providing a first web of material (NW1), preferably a liquid permeable nonwoven;
depositing absorbent material onto said first web at an absorbent-material-deposition zone (Z_{amd}) and such that one or more areas of said first web material (NW1) remain substantially void of absorbent material, said one or more areas corresponding to at least a plurality of discrete bonding regions (Bᵣ), and wherein the absorbent-material-deposition zone (Z_{amd}) comprises a first basis weight of absorbent material;
providing a second web of material (NW2), preferably a liquid permeable nonwoven, and applying said second web of material (NW2) over the deposited absorbent material, or folding said first web (NW1) to enclose the deposited absorbent material therein;
joining the first and second webs together, or the folded first web, at plurality of discrete bonding regions (Bᵣ) such that one or more unfastened regions (Uᵣ) are formed having a second basis weight of absorbent material;
optionally sandwiching the absorbent core between a liquid permeable layer and a substantially liquid impermeable layer oppositely disposed;
**characterized in that** the first basis weight is greater than the second basis weight and the plurality of discrete bonding regions (Bᵣ) are substantially connected to one or more unfastened regions (Uᵣ) with said discrete bonding regions (Bᵣ) being disposed outboard of a longitudinal centreline (y) of said absorbent core (4) such that said longitudinal centreline (y) is not substantially overlapped by said discrete bonding regions (Bᵣ) and **in that** the discrete bonding regions (Bᵣ) are positioned such that neighbouring bonding regions (Bᵣ) are separated by at least a portion of said absorbent-material-deposition zone (Z_{amd}) along a longitudinal direction (L_{d}) running substantially parallel to a longitudinal centreline of said absorbent core.

15. A method according to Claim 14 wherein the unfastened regions (Uᵣ) and the discrete bonding regions (Bᵣ) collectively form one or more channels (9).

16. A method according to any of Claims 14 to 15 comprising the step of applying a plurality of, preferably spaced apart, stripes of first adhesive on the second web of material (NW2), said stripes being offset from a centerline of said second web such that they overlap the discrete bonding regions (Bᵣ) and do not substantially overlap the one or more unfastened regions (Uᵣ).

## Amended claims

### Amended claims in accordance with Rule 137(2) EPC.

1. An absorbent article (1) comprising:
a topsheet (2);
a backsheet (3); and
an absorbent core (4) sandwiched between said topsheet (2) and backsheet (3);
said core (4) comprising absorbent material (5) that is substantially enclosed by a core wrap (6), wherein said core wrap (6) comprises a top core wrap layer (7) and a bottom core wrap layer (8), said core (4) comprising an absorbent-material-deposition zone (Z_{amd}) comprising a first basis weight of absorbent material (5), and wherein the top core wrap layer (7) and a bottom core wrap layer (8) are joined together at a plurality of discrete bonding regions (Bᵣ) substantially free of absorbent material; and wherein said core (4) further comprises one or more unfastened regions (Uᵣ) comprising a second basis weight of absorbent material (5),
and wherein the first basis weight is greater than the second basis weight; **characterised in that** the plurality of discrete bonding regions (Bᵣ) and the one or more unfastened regions (Uᵣ) are substantially connected to each other with a plurality of said discrete bonding regions (Bᵣ) being disposed outboard of a longitudinal centreline (y) of said absorbent core (4) and **in that** the discrete bonding regions (Bᵣ) are positioned such that neighbouring and/or consecutive bonding regions (Bᵣ) are separated by at least a portion of said absorbent-material-deposition zone (Z_{amd}) along a longitudinal direction (L_{d}) running substantially parallel to a longitudinal centreline (y) of said absorbent core (4).

2. An absorbent article (1) according to Claim 1 comprising a plurality of unfastened regions (Uᵣ) separated by discrete bonding regions (Bᵣ), preferably wherein said plurality of unfastened regions (Uᵣ) separated by discrete bonding regions (Bᵣ) are positioned in a front half and/or rear half of the absorbent core (4).

3. An absorbent article (1) according to any of the preceding Claims wherein the discrete bonding regions (Bᵣ) have an aspect ratio of from 0.4 to 3.0, preferably from 0.5 to 2.5, even more preferably of about 1.

4. An absorbent article (1) according to any of the preceding Claims wherein the discrete bonding regions (Bᵣ) are separated by one or more unfastened regions (Uᵣ) along an axis parallel to a transversal centerline (x) that is substantially perpendicular to the longitudinal centreline (y).

5. An absorbent article (1) according to any of the preceding Claims wherein the discrete bonding regions (Bᵣ) are separated by the absorbent-material-deposition zone (Z_{amd}) along at least an axis substantially parallel to the longitudinal centreline (y).

6. An absorbent article (1) according to any of the preceding Claims wherein ratio of first basis weight to second basis weight is greater than about 1.5, preferably greater than about 2, more preferably greater than 3, even more preferably greater than about 4, even more preferably greater than about 5.

7. An absorbent article (1) according to any of the preceding Claims wherein the top core wrap layer (7) or bottom core wrap layer (8) comprises a plurality of, preferably spaced apart, stripes of first adhesive (A1) extending along an axis substantially parallel to the longitudinal centreline (y), and wherein each said stripe overlaps with at least one, preferably at least two, of the plurality of discrete bonding regions (Bᵣ), and said stripes arranged such that each of said stripes does not substantially overlap with the one or more unfastened regions (Uᵣ), preferably wherein each said stripe overlaps at least a portion of the absorbent-material-deposition zone (Z_{amd}).

8. An absorbent article (1) according to Claim 7 wherein the opposing bottom core wrap layer (8) or top core wrap layer (7) is free of adhesive at least in areas that are congruent with the stripes of first adhesive.

9. An absorbent article (1) according to any of Claims 7 to 8 wherein the absorbent core comprises a left longitudinal half and right longitudinal half running along a length of said core and being adjacent to each other with the longitudinal centerline (y) being interposed therebetween, and wherein said plurality of stripes comprise at least two, preferably spaced apart, stripes that are positioned on said left longitudinal half and/or right longitudinal half.

10. An absorbent article (1) according to any of the preceding Claims wherein neighbouring bonding regions (Bᵣ) are free of unfastened regions (Uᵣ) in direct connection therewith along the longitudinal direction (L_{d}).

11. An absorbent article (1) according to any of the preceding Claims wherein the unfastened regions (Uᵣ) and the discrete bonding regions (Bᵣ) collectively form one or more channels (9) for guiding liquid via mass flow therethrough during a first time period, and wherein one or more portions of said channel(s) corresponding to the unfastened regions (Uᵣ) open to further accommodate absorbent material (5) therein as adjacent absorbent material (5) of the absorbent-material-deposition zone (Z_{amd}) swells.

12. An absorbent article (1) according to any of the preceding Claims wherein the core comprises unfastened regions (Uᵣ) for guiding liquid by capillary flow at least during a second time period.

13. An absorbent article (1) according to any of Claims 11 to 12 wherein the second time period occurs after the first time period; and/or wherein the second time period is greater than the first time period; and/or wherein the second time period is more than 15 seconds, preferably more than 20 seconds.

14. A method for the manufacture of absorbent articles, said method comprising the steps of:
providing a first web of material (NW1), preferably a liquid permeable nonwoven;
depositing absorbent material onto said first web at an absorbent-material-deposition zone (Z_{amd}) and such that one or more areas of said first web material (NW1) remain substantially void of absorbent material, said one or more areas corresponding to at least a plurality of discrete bonding regions (Bᵣ), and wherein the absorbent-material-deposition zone (Z_{amd}) comprises a first basis weight of absorbent material;
providing a second web of material (NW2), preferably a liquid permeable nonwoven, and
applying said second web of material (NW2) over the deposited absorbent material, or
folding said first web (NW1) to enclose the deposited absorbent material therein;
joining the first and second webs together, or the folded first web, at plurality of discrete bonding regions (Bᵣ) such that one or more unfastened regions (Uᵣ) are formed having a second basis weight of absorbent material;
optionally sandwiching the absorbent core between a liquid permeable layer and a substantially liquid impermeable layer oppositely disposed;
**characterized in that** the first basis weight is greater than the second basis weight and the plurality of discrete bonding regions (Bᵣ) are substantially connected to one or more unfastened regions (Uᵣ) with said discrete bonding regions (Bᵣ) being disposed outboard of a longitudinal centreline (y) of said absorbent core (4) such that said longitudinal centreline (y) is not substantially overlapped by said discrete bonding regions (Bᵣ) and **in that** the discrete bonding regions (Bᵣ) are positioned such that neighbouring bonding regions (Bᵣ) are separated by at least a portion of said absorbent-material-deposition zone (Z_{amd}) along a longitudinal direction (L_{d}) running substantially parallel to a longitudinal centreline of said absorbent core.

15. A method according to Claim 14 wherein the unfastened regions (Uᵣ) and the discrete bonding regions (Bᵣ) collectively form one or more channels (9).

16. A method according to any of Claims 14 to 15 comprising the step of applying a plurality of, preferably spaced apart, stripes of first adhesive on the second web of material (NW2), said stripes being offset from a centerline of said second web such that they overlap the discrete bonding regions (Bᵣ) and do not substantially overlap the one or more unfastened regions (Uᵣ).
